# EUROPEAN PATENT SPECIFICATION

(11) **EP 0 499 242 B1**
(45) Date of publication and mention of the grant of the patent: **26.06.1996**
(21) Application number: 92102387.5
(22) Date of filing: 13.02.1992
(51) Int. Cl.: A61K 38/00

(54) **Stimulation of endosteal bone formation with a cell growth factor**
Mit einem Zellwachstumsfaktor bewirkte Anregung von Wachstum im Knocheninneren
Stimulation de la formation endosseuse à l'aide d'un facteur de croissance cellulaire

(30) Priority: 15.02.1991 JP 44159/91; 20.09.1991 JP 270003/91
(43) Date of publication of application: 19.08.1992
(73) Proprietor: TAKEDA CHEMICAL INDUSTRIES, LTD., Chuo-ku, Osaka 541 (JP)
(72) Inventor: Mayahara, Hiroshi, Takatsuki, Osaka 569 (JP); Ito, Takayasu, Takatsuki, Osaka 569 (JP); Kato, Koichi, Kawanishi, Hyogo 666-01 (JP)
(74) Representative: von Kreisler, Alek, Dipl.-Chem.

(56) References cited:
- EP-A- 0 281 822
- EP-A- 0 298 723
- EP-A- 0 326 907
- EP-A- 0 394 951
- EP-A- 0 420 222
- EP-A- 0 421 455
- WO-A-87/01728
- BIOLOGICAL ABSTRACT no. 89055692; P. ASPENBERG et al.: "Fibroblast growth factor stimulates bone formation bone induction studied in rats" & Acta Orthopaedica Scandinavica 1989, vol. 60, no. 4, pages 473-476
- MEDLINE ABSTRACT no. 91130143; S. MOHAN et al. : "Bone growth factors" & Clin. Orthop. February 1991, no. 263, pages 30-48
- MEDLINE ABSTRACT no. 90373893; A.H. REDDI et al.: "Bone induction by osteogenin and bone morphogenetic proteins" & Biomaterials July 1990, no. 11, pages 33,34
- BIOLOGICAL ABSTRACT no. 89100871; P. MARIE et al. : "Effect of epidermal growth factor on bone formation and resorption in-vivo" & American Journal of Physiology 1990, vol. 258, no. 2, part 1, pages E275-281, 1990, vol. 258. no. 2. part 1. pages E275-281
- A Textbook of Histology, 11th ed., 1986, W.B. SAUNDERS Company, D.W. Fawsett, p. 206

## Description

### FIELD OF INVENTION

The present invention relates to stimulation of endosteal bone formation in warm-blooded animals.

### BACKGROUND OF THE INVENTION

Osteoporosis is a pathologic state characterized by pathologic bone mass reduction leading to clinical symptoms such as decrease in stature, and kyphosis leading to vertebral compression fracture and limb bone fracture. In developed countries, the number of osteoporosis patients has been increasing steadily with the aging of the population.

Bone mass reduction, the cause of osteoporosis, occurs due to various hormone abnormalities, particularly estrogen deficiency following menopause, a lack of calcitonin, growth hormone deficiency, hyperparathyroidism and hyperthyroidism as well as the physiological phenomenon of aging. Other factors affecting osteoporosis include diseases (chronic articular rheumatism and diabetes mellitus), nutritional or metabolic anomalies (deficiency of calcium, vitamin D, vitamin C or vitamin K, excessive intake of protein, phosphorus or sodium), the amount of exercise, heredity and interracial difference. Osteoporosis also occurs as a symptom (Cushing's syndrome) of the adverse effects of adrenocortical steroid, used to treat various inveterate diseases.

The bone tissue of adult mammals is constantly renewed (remodeling) in a well-balanced process of osteolysis/bone digestion (bone resorption) by osteoclasts and osteogenesis by osteoblasts. Osteoporosis can be considered as a state of constant bone mass reduction in which the amount of bone resorbed exceeds the amount of bone formed due to bone resorption and osteogenesis imbalance associated with various factors.

Traditionally, estrogen, calcitonin, active vitamin D, ipriflavone and other drugs have been administered to treat this disease.

Osteoporosis can be treated by administrating a drug which suppresses bone mass reduction or to increases bone mass. However, all the therapeutic drugs mentioned above mainly suppress bone resorption; there is no therapeutic drug possessing osteogenesis promoting action (bone mass increasing action) as an approved efficacy. Thus, often even long-term administration of these drugs to osteoporosis patients only delays progress of the symptoms. In addition, these drugs have almost no therapeutic value when osteoporosis has progressed to the extent that bone fracture occurs. Accordingly, there have been demands for the development of a drug with potent bone mass increasing action or osteogenesis promoting action in short-term administration.

Taking note of the background situation described above, the present inventors made investigations and found that cell growth factors stimulate endosteal bone formation in warm-blooded animals. The inventors made further investigations based on this finding and completed the present invention.

Thus, the present invention is directed to stimulation of endosteal bone formation in warm-blooded animals.

### BRIEF DESCRIPTION OF THE DRAWINGS:

Figure 1 is a light microscopic photomicrograph showing a histologic image of the femur in the untreated control group, obtained in Example 1.

Figure 2 is a light microscopic photomicrograph showing a histologic image of the femur in the group treated with rhbFGF mutein CS23, obtained in Example 1.

Figure 3 is a light microscopic photomicrograph showing another histologic image of the femur in the untreated control group, obtained in Example 1.

Figure 4 is a light microscopic photomicrograph showing another histologic image of the femur in the group treated with rhbFGF mutein CS23, obtained in Example 1.

Figure 5 is a light microscopic photomicrograph showing a histologic image of the sternum in the untreated control group, obtained in Example 1.

Figure 6 is a light microscopic photomicrograph showing a histologic image of the sternum in the group administered with rhbFGF mutein CS23, obtained in Example 1.

Figure 7 is a light microscopic photomicrograph showing a histologic image of the femur in the untreated control group, obtained in Example 2.

Figure 8 is a light microscopic photomicrograph showing a histologic image of the femur in the rhbFGF administration group, obtained in Example 2.

Figure 9 is a light microscopic photomicrograph showing a histologic image of the femur in the untreated control group, obtained in Example 3.

Figure 10 is a light microscopic photomicrograph showing a histologic image of the femur in the rhaFGF administration group, obtained in Example 3.

Figure 11 is a light microscopic photomicrograph showing a histologic image of the femur in the untreated control group, obtained in Example 4.

Figure 12 is a light microscopic photomicrograph showing a histologic image of the femur in the hst-1 mutein N27 administration group, obtained in Example 4.

Figure 13 is a light microscopic photomicrograph showing a histologic image of the femur in the untreated control group, obtained in Example 5.

Figure 14 is a light microscopic photomicrograph showing a histologic image of the femur in the EGF administration group, obtained in Example 5.

Figure 15 is a light microscopic photomicrograph showing a histologic image of the tibia in the untreated control group, obtained in Example 6.

Figure 16 is a light microscopic photomicrograph showing a histologic image of the tibia in the rhbFGF mutein CS23 administration group, obtained in Example 6.

Figure 17 is a contact microradiogram (CMR) of the tibia in the untreated control group, obtained in Example 6.

Figure 18 is a CMR of the tibia in the rhbFGF mutein CS23 administration group, obtained in Example 6.

Figure 19 is a light microscopic photomicrograph showing a histologic image of the femur in the untreated control group, obtained in Example 7.

Figure 20 is a light microscopic photomicrograph showing a histologic image of the femur in the rhbFGF mutein CS23 administration group, obtained in Example 7.

### SUMMARY OF THE INVENTION

The present invention provides (1) a method of stimulation of endosteal bone formation of a warm-blooded animal, which comprises administering an effective amount of a cell growth factor to said animal;
(2) a pharmaceutical cell growth factor composition to provide a prophylactic or therapeutic action for the stimulation of endosteal bone formation of warm-blooded animals, comprising [A] an effective amount of a cell growth factor and [B] a pharmaceutically acceptable carrier, diluent or excipient;
(3) use of a cell growth factor composition to produce a medicament to provide a prophylactic or therapeutic action for the stimulation of endosteal bone formation of a warm-blooded animal; and
(4) a method of producing a medicament to provide a prophylactic or therapeutic action for the stimulation of endosteal bone formation in warm-blooded animals, which comprises admixing an effective amount of a cell growth factor with a pharmaceutcally acceptable carrier, diluent or excipient.

### DETAILED DESCRIPTION OF THE INVENTION

As the cell growth factors for the present invention is exemplified by those having a molecular weight of 5000 to 25000.

Examples of the cell growth factors include a peptide belonging to the fibroblast growth factor (FGF) family, nerve growth factors NGF-1 and NGF-2/NT-3, epithelial growth factor (EGF), platelet-derived growth factor (PDGF), insulin-like growth factor (IGF)-I and -II, colony stimulating factor (CSF), erythropoietin (EPO), thrombopoietin, interleukins IL-1, IL-2, IL-3, IL-4, IL-5 and IL-6, cartilage-derived factor (CDF), transforming growth factors TGF-α, TGF-β and TGF-γ2, tumor angiogenesis factor (TAF), insulin, transferrin, fibronectin, laminin, chondronectin, hydronectin, hepatocyte growth factor (HGF), leukemia cell-derived growth factor (LGF), macrophage growth factor (MGF), bone morphogenetic protein (BMP)-1, -2A, -2B and -3, B2 microglobulin (B2-m), and osteoinductive factor (OIF). These cell growth factors may be of the naturally occurring type or of the mutein type.

Preference is given to a peptide which belongs to the FGF family.

The FGF family includes a basic FGF (hereinafter also referred to as bFGF), acidic FGF (hereinafter also referred to as aFGF), int-2, hst-1, k-FGF, FGF-5, FGF-6 and so forth. Muteins thereof are also included.

The FGF may be derived from mammals. Examples of such mammals include humans, monkeys, swine, bovines, sheep and horses.

The FGF may also be extracted from various organs known to contain FGF, such as the brain and the pituitary gland. The FGF may also be produced by gene engineering techniques.

The bFGF may be obtained by recombinant DNA technology (FEBS Letters, *213*, 189-194 (1987); European Patent Application Publication No. 237,966).

In the present specification, recombinant human basic FGF is also referred to as rhbFGF.

The FGF may be a mutein. The FGF mutein is essentially a variant resulting from amino acid sequence mutation in the original peptide or protein; therefore, such mutations include amino acid addition, constitutional amino acid deletion and substitution by other amino acids.

Said amino acid addition means addition of at least one amino acid.

Said constitutional amino acid deletion means deletion of at least one FGF-constituting amino acid.

Said substitution by other amino acids means substitution of at least one FGF-constituting amino acid by another amino acid.

Said amino acid addition includes neither methionine originating from the initiation codon for peptide expression nor signal peptide.

In producing the above muteins via amino acid addition, any number of amino acids may be added, as long as the nature of FGF is maintained. Preferably, the amino acid added may be a partial or entire amino acid sequence of any protein showing homology to FGF and hence similar activities.

In producing the above muteins via constitutional amino acid deletion from FGF, any number of amino acids may be lacking, as long as the nature of FGF is maintained.

In producing the above muteins via substituion by other amino acids, any number of amino acids may be substituted by other amino acids, as long as the nature of FGF is maintained. The substituted mutein is preferably a mutein wherein at least one constituent amino acid cysteine has been substituted by serine. In said substitution, two or more substitutions may be carried out at the same time, while substitution of two or three constituent amino acids is preferred.

The mutein may result from a combination of two or three additions, deletions, and substitutions as described above or some combination thereof. The mutein may also result from introduction of a sugar chain binding site.

The mutein is exemplified by the muteins described in European Patent Application (hereinafter also referred to as EP) Publication No. 281,822, No. 326,907 and No. 394,951.

The mutein is preferably a mutein resulting from substitution of at least one human basic FGF-constituting amino acid by another amino acid, with preference given to human bFGF mutein CS23, resulting from substitution of the 70- and 88-Cys residues by Ser (serine) in human bFGF (see EP No. 281,822).

The aFGF described above is exemplified by the FGF having the amino acid sequence shown in Biochemical and Biophysical Research Communications, *138,* 611-617 (1986).

Typical aFGF muteins include deletion type muteins such as those comprising a polypeptide chain of 90 to 133 continuous amino acids as a part of the sequence of aFGF comprising 140 amino acids, with preference given to muteins comprising a polypeptide chain of 120 to 131 continuous amino acids as a part of the amino acid sequence of aFGF.

Examples of such aFGF muteins include those described in EP Publication No. 420,222.

The hst-1 described above is exemplified by the proteins disclosed in the Proceedings of National Academy of Science, USA, *84*, 2980 (1987), the Proceedings of National Academy of Science, USA, *84,* 7305 (1987) and Molecular and Cellular Biology, *8*, 2933 (1988).

Examples of hst-1 muteins include deletion type muteins such as those resulting from deletion of at least one of the 175 hst-1-constituting amino acids, preferably from deletion of 1 to 47 continuous hst-1-constituting amino acids, more preferably from deletion of 1 to 43 continuous hst-1 constituting amino acids, and best is from deletion of any 1 to 27 continuous hst-1-constituting amino acids.

Examples of such hst-1 muteins include those described in EP Publication No. 421,455.

The EGF for the present invention may be prepared by gene recombination technology, for instance. Examples of such production methods include the method described in EP Publication No. 326,046.

In the present invention, endosteal bone formation is stimulated. In particular, when the present medicament is administered to a warm-blooded animal, osteoblasts proliferate markedly, new bone formation occurs markedly and bone salt mass increases markedly in all the long or pipe bones such as the femurs, in the flat bones such as the sternum and ribs and in the irregular bones such as the vertebrae, according to the route, dosage and the period of administration. Its toxicity is low. Therefore, the present medicament can be used as a prophylactic/therapeutic medicine for various bone diseases including bone-loss diseases such as osteoporosis and so forth.

In addition, there is provided in the present invention a method whereby the osteoblast proliferation and bone formation occur on the endosteum side of the diaphysis and metaphysis, and no bone formation occurs on the periosteum side (outer surface) of the bone. Therefore, the medicament of the present invention can stimulate the endosteal bone formation, and new bone is formed towards the marrow cavity. The endosteal bone formation increases the thickness of the cortical bone of the diaphysis. It also increases the thickness of each trabecular bones in the cancellous (spongy) bone in the metaphysis. Both the increase in the cortical bone thickness in the diaphysis and the increase in the thickness of each trabecular bones in the metaphysis reinforce the skeletal bones, and thus prevent or assist in the therapy of the bone-loss diseases such as osteoporosis, osteopenia, hypercalcemia and so forth.

The nature of the medicament of the present invention, which is stimulation of the endosteal bone formation but not the periosteal (outside) bone formation, is best suited for prophylactic/therapeutic action against bone-loss diseases because it can increase bone mass without the disadvantages of pain or skeletal deformation due to outward extension of the bone tissue.

In mature animals, old bone tissue is first resorbed by osteoclasts (bone resorption) and then repaired by osteoblasts (osteogenesis), thus maintaining bone homeostasis; this mechanism is called remodeling. In the present invention, the bone formation caused by cell growth factor is accompanied by osteoblast proliferation, but not osteoclast proliferation. In other words, the present invention provides for bone formation without homeostatic remodeling. Previous drug treatments for bone-loss diseases sought to decrease bone loss by decreasing bone resorption by osteoclasts. Therefore, the bone mass increase effect of the previous drug treatment is indirect and slow, if any. In contrast, the bone formation by the cell growth factor of the present invention is direct and quick because it stimulates osteoblast proliferation and osteogenesis directly, without interfering the process of remodeling.

Examples of target bone diseases in the present invention, whether congenital or acquired, include bone-loss diseases such as osteoporosis, osteopenia, osteodystrophy, dysostosis, and hypercalcemia. The prophylactic/therapeutic medicament for bone diseases of the present invention is very effective in the prevention or treatment of bone diseases involving bone mass reduction and bone tissue cavitation, such as osteoporosis, because it causes osteoblast proliferation, increase in new bone formation and increase in bone salt mass and in turn reinforces all the long or pipe bones, flat bones and irregular bones.

The preventive/therapeutic medicament for bone diseases of the present invention can be safely administered orally or parenterally as such or in the form of pharmaceutical compositions along with a pharmacologically acceptable carrier, e.g. carriers, excipients or diluents for, injections, tablets, capsules, solutions, ointments and so forth to warm-blooded animals (e.g., humans, mice, rats, hamsters, rabbits, dogs, cats, bovines, sheep, swine, horses), preferably mammals, for the purpose of preventing or treating their bone diseases, with preference given to parenteral administration.

As for dosage form, it is preferable to prepare the medicament of the invention as an injection, a solution for injection, or a lyophilized product. It may also be prepared as a sustained-release preparation as necessary.

In preparing the medicament of the present invention as a pharmaceutical composition, known pharmaceutical production procedures are followed, using pharmaceutically acceptable carriers, diluents or excipients as necessary.

When preparing the medicament as an aqueous solution for injection, for instance, it is produced by conventional methods using aqueous solvents (e.g., distilled water), water-soluble solvents (e.g., physiological saline, Ringer's solution) and oily solvents (e.g., sesame oil, olive oil), with dissolution aids (e.g., sodium salicylate, sodium acetate), buffers (e.g., sodium citrate), isotonizing agents (e.g., glucose, invertose), stabilizers (e.g., human serum albumin, polyethylene glycol), preservatives (e.g., benzyl alcohol, phenol), analgesics (e.g., benzalkonium chloride, procaine hydrochloride) and other additives added as necessary.

When preparing the medicament of the present invention as a solid preparation for injection, for instance, the desired solid preparation for injection can be produced by conventional methods using a mixture of diluents (e.g., distilled water, physiological saline, glucose), excipients (e.g., carboxymethyl cellulose (CMC), sodium alginate), preservatives (e.g., benzyl alcohol, benzalkonium chloride, phenol), analgesics (e.g., glucose, calcium gluconate, procaine hydrochloride) or other substances.

In producing a pharmaceutical preparation for the medicament of the present invention, monosaccharides such as glucose, amino acids, various salts, human serum albumin and other substances may be added, with isotonizing medicaments, pH regulators, analgesics, antiseptics and other additives added to yield stable, effective preparations.

Concerning the dose of the medicament of the present invention, it should be administered in such amounts that the cell growth factor content in the body fluid of living bodies is about 1 to about 1000 pg/ml, preferably about 10 to about 500 pg/ml, corresponding to about 0.1 to about 1000 µg/kg body weight, preferably about 1 to about 300 µg/kg body weight.

Routes of parenteral administration of the medicament of the present invention include intravenous administration, subcutaneous administration, intramuscular administration, injection directly into the marrow cavity or bone tissue and permucous administration. Routes of mucous membrane administration include nasal administration, intraoral administration and rectal administration.

It is preferable that the medicament of the present invention be administered so that it is delivered systemically to the warm-blooded animal subject. It is therefore preferable to administer it into the circulatory system, for instance, by intravenous administration. The administration frequency may range from more than once a day to less than once a week, and may be regular or intermittent.

The medicament of the present invention may also be administered in the form of a sustained-release preparation. Such sustained-release preparations include microcapsules and implant pellets. In particular, a subcutaneously-implanted sustained-release preparation insures a sustained effect of the active ingredient. In a preferred embodiment of the present invention, the sustained-release preparation is implanted or injected directly in the medullary cavity or bone tissue insuring a sustained release of the active ingredient.

In administering the preparation according to the present invention, a calcium medicament may be used concomitantly, since osteogenesis requires calcium. Bone resorption suppressors such as active vitamin D₂, active vitamin D₃, calcitonin, estrogen and ipriflavone may also be used concomitantly.

The recombinant human basic FGF mutein CS23 (hereinafter also referred to as rhbFGF mutein CS23) used in Examples given below is produced by the method described in Examples 7 and 24 for EP Publication No. 281,822 and Biochemical and Biophysical Research Communications, *151,* 701-708 (1988), employing the transformant *Escherichia coli* MM294/pTB762 (IFO 14613, FERM BP-1645).

The above-mentioned transformant *E*. *coli* MM294/pTB762 has been deposited at the Institute for Fermentation, Osaka, Japan and at the Fermentation Research Institute, Agency of Industrial Science and Technology, Ministry of International Trade and Industry of Japan. The accession numbers and dates of accession are shown in Table 1 below. With respect to the FRI deposition, a domestic deposition was first made under a FERM P accession number. This deposition was then converted to a deposition under the Budapest Treaty; since then the transformant has been stored at FRI under a FERM BP accession number.

**Table 1**

| Transformant | IFO | FRI |
|---|---|---|
| _{*E. coli*} MM294/pTB762 | IFO 14613 (May 27, 1987) | FERM P-9409 FERM BP-1645 (June 11, 1987) |

The human bFGF used in Examples given below was produced by the method described in Examples of EP Publication No. 237,966 using the transformant *Escherichia coli* K12MM294/pTB669 (IFO 14532, FERM BP-1281).

The human aFGF used in Examples given below was produced by the method described in Example 1 of EP Publication No. 406,738 using the transformant *Escherichia coli* MM294(DE3)/pLysS,pTB975 (IFO 14936, FERM BP-2599).

The hst-1 mutein N27 used in Examples given below was produced by the method described in Examples of EP Publication No. 421,455 using the transformant *Escherichia coli* MM294(DE3)/pLysS,pTB1051 (IFO 14952, FERM BP-2621).

The EGF used in Examples given below was produced by the method described in EP Publication No. 326,046. Specifically, the EGF was produced by the method described in Examples 1, 2, 3 (ii), 4 and 5 for EP Publication No. 326,046 using *Bacillus brevis* H102(pNU200-EGF31), the transformant was prepared by inserting the plasmid pNU200-EGF31, separated from the transformant *Bacillus brevis* 47-5(pNU200-EGF31) (IFO 14729, FERM BP-1772), to the host *Bacillus brevis* H102 (FERM BP-1087, see U.S. Patent 4,946,789).

### EXAMPLES

The present invention is hereinafter described in more detail by means of the following reference example and working examples, but the invention is not by any means limited thereby.

### Reference Example 1 Method of measuring bone formation or osteogenesis

A cross-sectional specimen of femur or tibia was taken from a site 5 to 10 mm from the distal end. An X-ray radiogram was taken of a sagittal section of the specimen, using a soft X-ray radiographic apparatus (Softex, model CSM-2). On this X-ray radiogram were determined the cross sectional area (A) and medullary cavity area (B) of the specimen, using an image analyzer (Pias Corporation, model 555). Value B was subtracted from value A to obtain the cortical bone area, and was expressed in percent ratio to value A.

### Example 1 Osteogenesis promoting action of rhbFGF mutein CS23 in rats

Four male rats and four female rats (Jcl: Wistar) in each group were dosed with rhbFGF mutein CS23 at 0.01, 0.03, 0.1 or 0.3 mg/kg/day by intravenous administration for 2 weeks starting at 6 weeks of age. Upon completion of the administration period, the animals were killed, and their femurs, sterna and ribs were fixed in 10% formalin, decalcified embedded in paraffin and sliced longitudinally to yield 4-micron sections. After staining with hematoxylin-eosin (H-E), these sections were examined histopathologically to assess the effects on bone tissue. Animals in the control group were dosed with physiological saline in the same manner as of the treated groups.

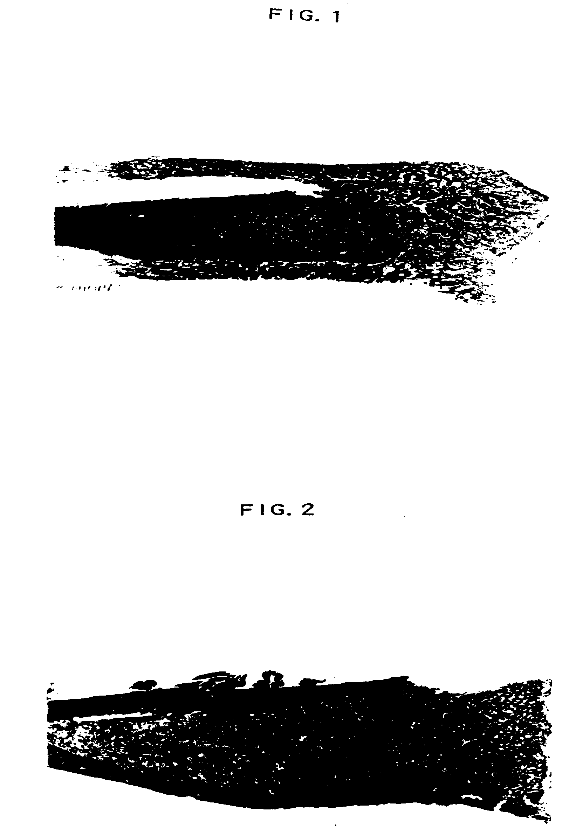

As a result, osteoblast proliferation and were seen dose-dependently at doses of 0.1 mg/kg or more in the femurs, sterna and ribs. This finding was found in the marrow cavity side of the metaphysis and diaphysis in the femurs (see Figures 1, 2, 3 and 4), and in the marrow cavity side of the central portion of bone in the sterna and ribs.

Details of Figures 1 through 4 are as follows:
Figure 1: Femur, Jcl: Wistar rat, 8-week-old female, untreated control group, H-E stained, × 8.5, no abnormal findings.
Figure 2: Femur, Jcl: Wistar rat, 8-week-old female, rhbFGF mutein CS23 0.3 mg/kg group, H-E stained, × 8.5, showing new bone formation in the marrow cavity.

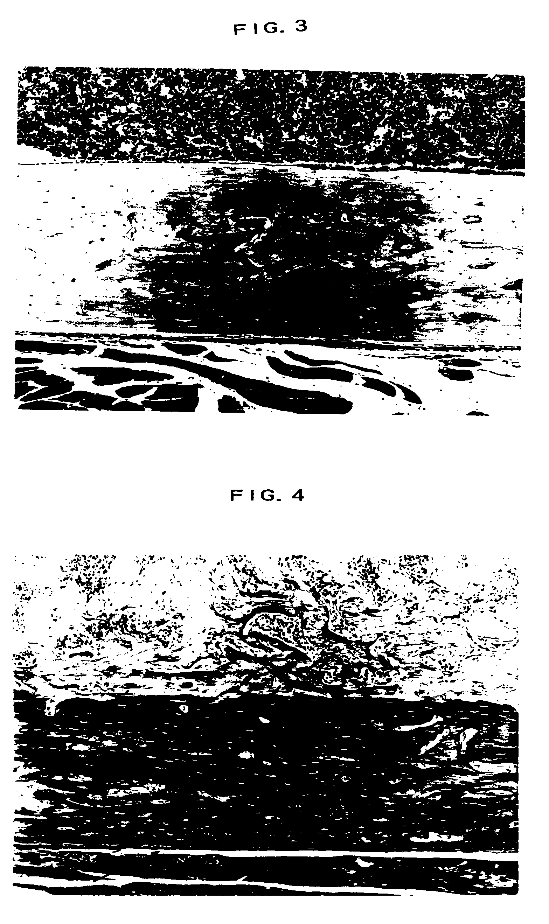

Figure 3: Femur, Jcl: Wistar rat, 8-week-old female, untreated control group, H-E stained, × 85, no abnormal findings.
Figure 4: Femur, Jcl: Wistar rat, 8-week-old female, rhbFGF mutein CS23 0.3 mg/kg group, H-E stained, × 85, showing new bone formatioin in the marrow cavity, no osteogenesis is seen on the periosteum side (arrow).

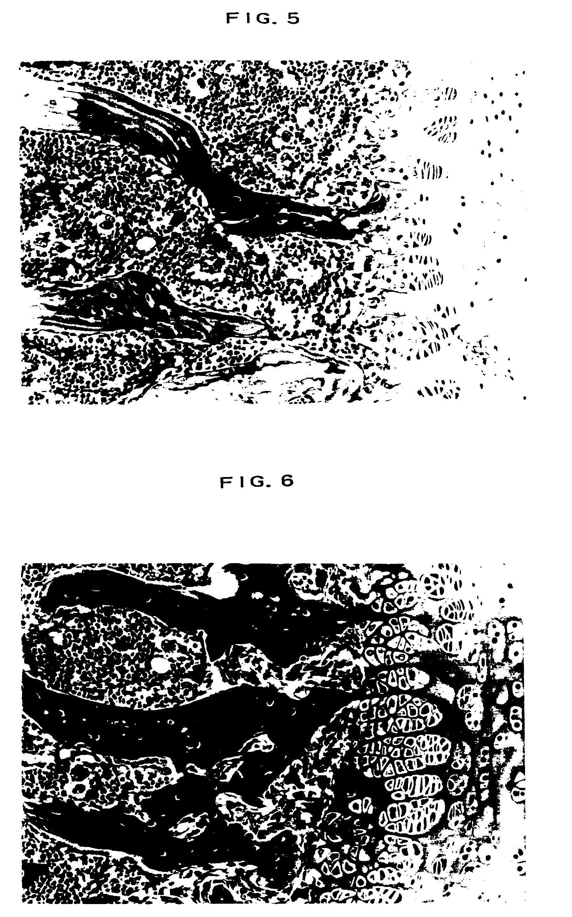

In the 0.3 mg/kg group, similar findings were obtained on the marrow cavity side of the ribs at the costochondral joint. In these bones, osteogenesis occurred on the marrow cavity side alone and did not occur on the outer side (periosteum side) of the bone. No osteoclast proliferation occurred at any dose level. Also noted was epiphysial osteoblast proliferation in the 0.3 mg/kg administration group, indicating promotion of endochondral bone formation (see Figures 5 and 6).

Details of Figures 5 and 6 are as follows:
Figure 5: Sternum, Jcl: Wistar rat, 8-week-old female, untreated control group, H-E stained, × 170, no abnormal findings.
Figure 6: Sternum, Jcl: Wistar rat, 8-week-old female, rhbFGF mutein CS23 0.3 mg/kg group, H-E stained, × 170, showing hypertrophy and proliferation of the osteoblasts (arrow) and new bone formation.

In accordance with the method described in Reference Example 1, cortical bone area (%) was calculated on the femur specimens from the 0.3 mg/kg administration group (results are given in Table 2). The cortical bone area (%) in the 0.3 mg/kg administration group was statistically greater than that in the physiological saline administration group.

**Table 2**

| Group | Number of animals | Cortical bone area (%) |
|---|---|---|
| Physiological saline administration group | 4 | 59.6 ± 2.4 |
| rhbFGF mutein CS23 0.3 mg/kg administration group | 4 | 70.2 ± 2.1* |

| | | |
|---|---|---|
| *: Indicates that the difference between the groups is statistically significant at a significance level of 1% (mean ± SE). | | |

### Example 2 Osteogenesis promoting action of rhbFGF in rats

Four female rats (Jcl: Wistar) in each group were dosed with human basic fibroblast growth factor (rhbFGF) at 0.03, 0.1 or 0.3 mg/kg/day by intravenous administration for 2 weeks starting at 6 weeks of age. One day after final administration, the animals were killed, and the femurs, sterna and ribs were fixed in 10% formalin, decalcified, embedded in paraffin and sliced longitudinally to yield 4-micron sections. After staining with hematoxylin-eosin (H-E), these sections were examined histopathologically to assess the effects on bone tissue. Animals in the control group were dosed with 0.02 M Tris-HCl-1M NaCl buffer (pH 7.4) in the same manner as of the treated groups.

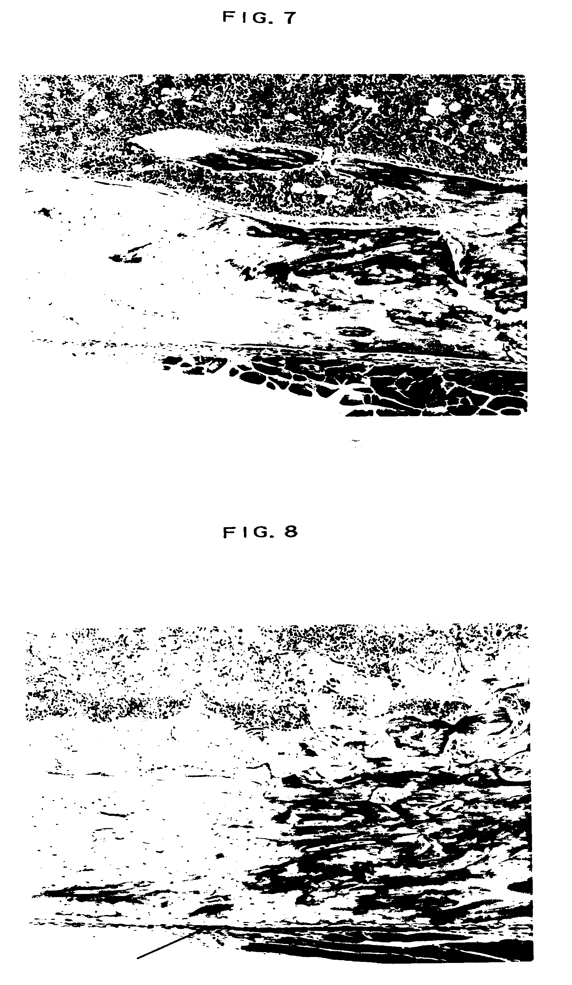

As a result, osteoblast proliferation and new bone formation were seen dose-dependently at doses of 0.03 mg/kg or more in the femurs and at 0.1 mg/kg or more in the sterna. This finding was obtained from the marrow cavity side of the metaphysis and diaphysis in the femurs (see Figures 7 and 8), and from the marrow cavity side of the central portion of the sterna. No osteogenesis occurred on the periosteum side.

Details of Figures 7 and 8 are as follows:
Figure 7: Femur, Jcl: Wistar rat, 8-week-old female, untreated control group, H-E stained, × 85, no abnormal findings.
Figure 8: Femur, Jcl: Wistar rat, 8-week-old female, rhbFGF 0.3 mg/kg group, H-E stained, × 85, showing new bone formation in the marrow cavity, no new bone formation is seen on the periosteum side (arrow).

In accordance with the method described in Reference Example 1, cortical bone area (%) was calculated on the femur specimens from the 0.3 mg/kg administration group (results are given in Table 3). The cortical bone area (%) in the 0.3 mg/kg administration group was statistically greater than that in the Tris-HCl buffer-administration group.

**Table 3**

| Group | Number of animals | Cortical bone area (%) |
|---|---|---|
| 0.02 M Tris-HCl buffer administration group | 4 | 43.6 ± 0.9 |
| rhbFGF 0.3 mg/kg administration group | 4 | 52.0 ± 1.3* |

| | | |
|---|---|---|
| *: Indicates that the difference between the groups is statistically significant at a significance level of 1% (mean ± SE). | | |

### Example 3 Osteogenesis promoting action of rhaFGF in rats

Four female rats (Jcl: Wistar) in each group were dosed with human acidic fibroblast growth factor (rhaFGF) at 0.03, 0.1 or 0.3 mg/kg/day by intravenous administration for 2 weeks starting at 6 weeks of age. One day after final administration, the animals were killed, and their femurs, sterna and ribs were fixed in 10% formalin, decalcified, embedded in paraffin and sliced longitudinally to yield 4-micron sections. After staining with hematoxylin-eosin (H-E), these sections were examined histopathologically to assess the effects on bone tissue. Animals in the control group were dosed with Tris-HCl-0.9M NaCl buffer (pH 7.4) in the same manner as of the treated groups.

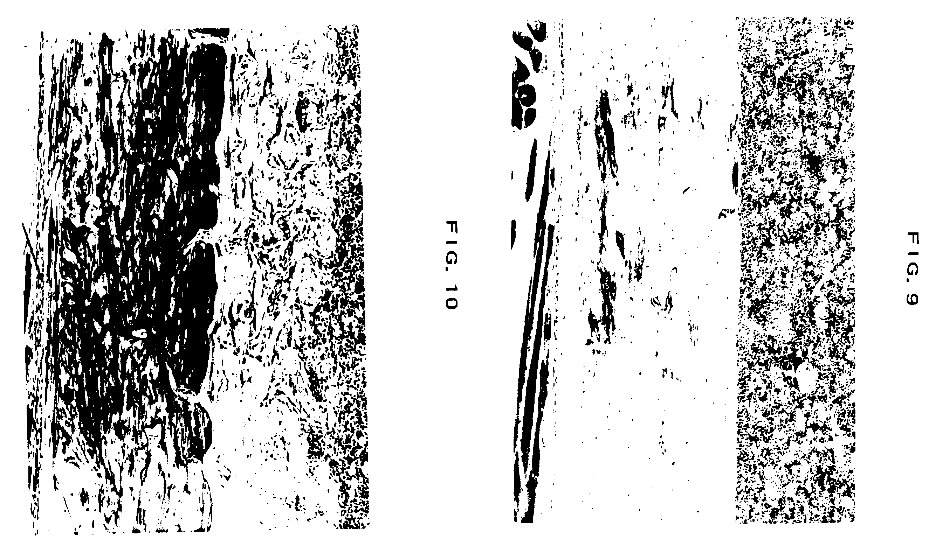

As a result, osteoblast proliferation and new bone formation were seen dose-dependently at doses of 0.03 mg/kg or more in the femurs and sterna. This finding was obtained from the marrow cavity side of the metaphysis and diaphysis in the femurs (see Figures 9 and 10), and from the marrow cavity side of the central portion of the sterna. No osteogenesis was seen on the periosteum side.

### Details of Figures 9 and 10 are as follows:

Figure 9: Femur, Jcl: Wistar rat, 8-week-old female, untreated control group, H-E stained, × 85, no abnormal findings.
Figure 10: Femur, Jcl: Wistar rat, 8-week-old female, rhaFGF 0.3 mg/kg group, H-E stained, × 85, showing new bone formation in the marrow cavity, no osteogenesis is seen in the periosteum side (arrow).

In accordance with the method described in Reference Example 1, cortical bone area (%) was calculated on the femur specimens from the 0.1 mg/kg administration group (results are given in Table 4). The cortical bone area (%) in the 0.1 mg/kg administration group was statistically greater than that in the Tris-HCl buffer-administration group.

**Table 4**

| Group | Number of animals | Cortical bone area (%) |
|---|---|---|
| 0.02 M Tris-HCl buffer administration group | 4 | 43.9 ± 0.6 |
| rhaFGF 0.1 mg/kg administration group | 4 | 48.7 ± 1.8* |

| | | |
|---|---|---|
| *: Indicates that the difference between the groups is statistically significant at a significance level of 1% (mean ± SE). | | |

### Example 4 Osteogenesis promoting action of hst-1 mutein N27 in rats

Four female rats (Jcl: Wistar) in each group were dosed with recombinant human heparin-binding secretory transforming protein-1 (hst-1) mutein N27 at 0.03, 0.1 or 0.3 mg/kg/day by intravenous administration for 2 weeks starting at 6 weeks of age. One day after final administration, the animals were killed, and the femurs, sterna and ribs were fixed in 10% formalin, decalcified, embedded in paraffin and sliced longitudinally to yield 4-micron sections. After staining with hematoxylin-eosin (H-E), these sections were examined histopathologically to assess the effects on bone tissue. Animals in the control group were dosed with 0.01M Tris-HCl-0.2 M NaCl buffer (pH 7.4) in the same manner as of the treated groups.

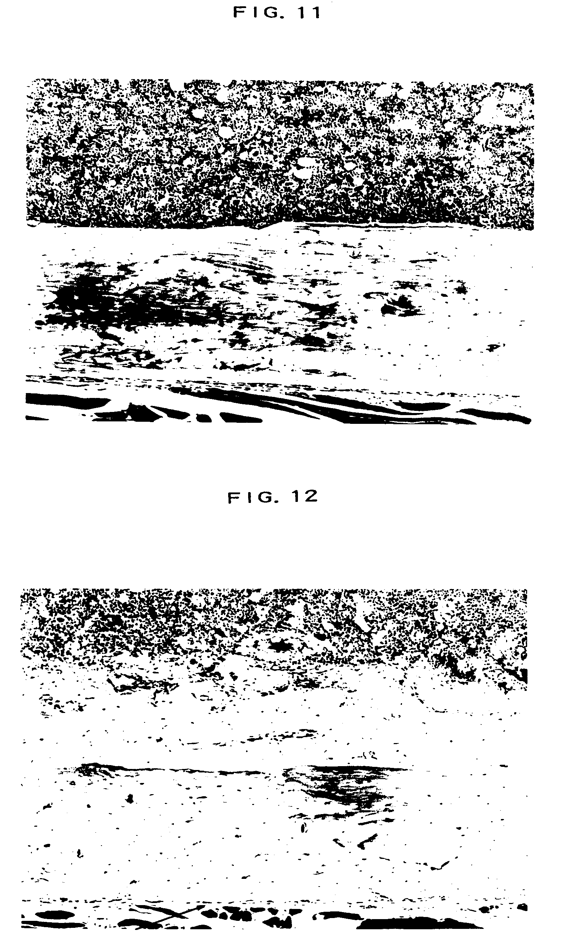

As a result, osteoblast proliferation and new bone formation were seen dose-dependently at doses of 0.03 mg/kg or more in the femurs and sterna and at 0.1 mg/kg or more in the ribs. This finding was obtained from the marrow cavity side of the metaphysis and diaphysis in the femurs (see Figures 11 and 12), and from the marrow cavity side of the central portion of the sterna and ribs. No osteogenesis was seen on the periosteum side.

Details of Figures 11 and 12 are as follows:
Figure 11: Femur, Jcl: Wistar rat, 8-week-old female, untreated control group, H-E stained, × 85, no abnormal findings.
Figure 12: Femur, Jcl: Wistar rat, 8-week-old female, hst-1 mutein N27 0.3 mg/kg group, H-E stained, × 85, showing new bone formation in the medullary cavity, no new bone formation is seen on the periosteum side (arrow).

In accordance with the method described in Reference Example 1, cortical bone area (%) was calculated on the femur specimens from the 0.1 mg/kg administration group (results are given in Table 5). The cortical bone area (%) in the 0.1 mg/kg administration group was statistically greater than that in the Tris-HCl buffer-administration group.

**Table 5**

| Group | Number of animals | Cortical bone area (%) |
|---|---|---|
| 0.01M Tris-HCl buffer administration group | 4 | 46.4 ± 1.2 |
| hst-1 mutein N27 0.1 mg/kg administration group | 4 | 52.9 ± 2.3* |

| | | |
|---|---|---|
| *: Indicates that the difference between the groups is statistically significant at a significance level of 1% (mean ± SE). | | |

### Example 5 Osteogenesis promoting action of EGF in rats

Four female rats (Jcl: Wistar) in each group were dosed with epithelial growth factor (EGF) at 0.03, 0.1 or 0.3 mg/kg/day by intravenous administration for 2 weeks starting at 6 weeks of age. One day after final administration, the animals were killed, and the femurs, sterna and ribs were fixed in 10% formalin, decalcified, embedded in paraffin and sliced longitudinally to yield 4-micron sections. After staining with hematoxylin-eosin (H-E), these sections were examined histopathologically to assess the effects on bone tissue. Animals in the control group were dosed with ammonium acetate buffer (pH 6.8) in the same manner as of the treated groups.

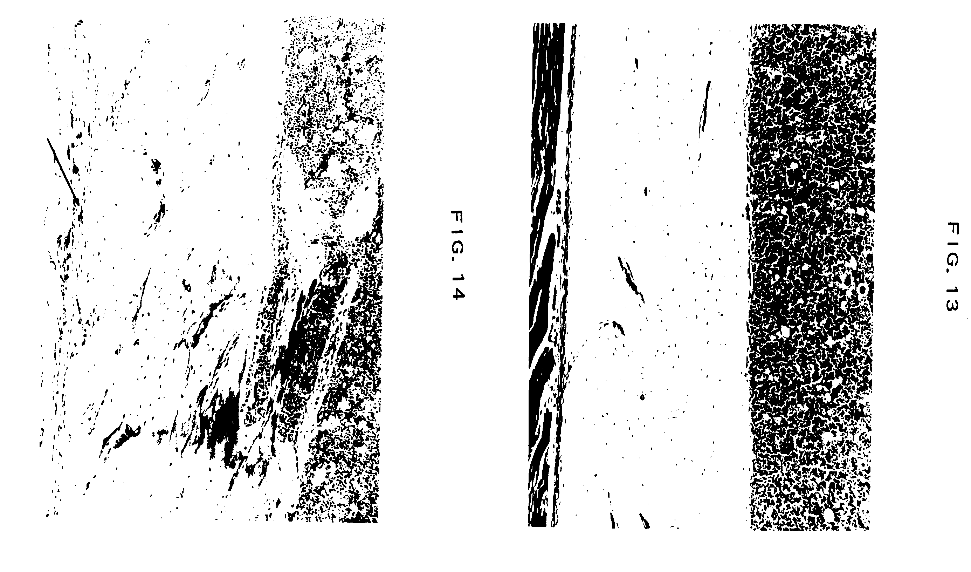

As a result, osteoblast proliferation and new bone formation were seen dose-dependently at doses of 0.1 mg/kg or more in the femurs. This finding was obtained from the marrow cavity side of the metaphysis and diaphysis in the femurs (see Figures 13 and 14). No osteogenesis was seen on the periosteum side.

Details of Figures 13 and 14 are as follows:
Figure 13: Femur, Jcl: Wistar rat, 8-week-old female, untreated control group, H-E stained, × 85, no abnormal findings.
Figure 14: Femur, Jcl: Wistar rat, 8-week-old female, EGF 0.3 mg/kg group, H-E stained, × 85, showing new bone formation in the marrow cavity, no osteogenesis is seen on the periosteum side (arrow).

In accordance with the method described in Reference Example 1, cortical bone area (%) was calculated on the femur specimens from the 0.3 mg/kg administration group (results are given in Table 6). The cortical bone area (%) in the 0.3 mg/kg administration group was statistically greater than that in the ammonium acetate buffer-administration group.

**Table 6**

| Group | Number of animals | Cortical bone area (%) |
|---|---|---|
| Ammonium acetate buffer administration group | 4 | 57.0 ± 2.0 |
| EGF 0.3 mg/kg administration group | 4 | 66.1 ± 1.3* |

| | | |
|---|---|---|
| *: Indicates that the difference between the groups is statistically significant at a significance level of 1% (mean ± SE). | | |

### Example 6 Calcified cancellous bone formation promoting action of rhbFGF mutein CS23 in rats

Five male rats (Jcl: Wistar) were dosed with rhbFGF mutein CS23 at 0.1 mg/kg/day by intravenous administration for 2 weeks starting at 6 weeks of age. Five animals in the control group were dosed with a solvent for dissolution of rhbFGF mutein CS23 (50 mM citrate buffer) in the same manner as with the treated group. Upon completion of the administration period, animals were killed by exsanguination, and the tibias were fixed in 70% ethanol. A proximal portion of the tibia was cut using a bone cutter, stained with Vilanueba Bone (VB), dehydrated and then embedded in methyl methacrylate resin. Two hundred and fifty micron thin frontal sections were cut out from the resin-embedded tibia using a bone slicer and polished to 70 micron thickness using a mechanical polisher. From these polished specimens, contact microradiograms (CMR) were taken using a soft X-ray radiographic apparatus. Films were developed with D-19, stopped, fixed and then air dried and embedded in Canada balsam to yield microscopic specimens. Polished specimens were further polished to 50 micron thickness, and embedded in Canada balsam to yield microscopic specimens.

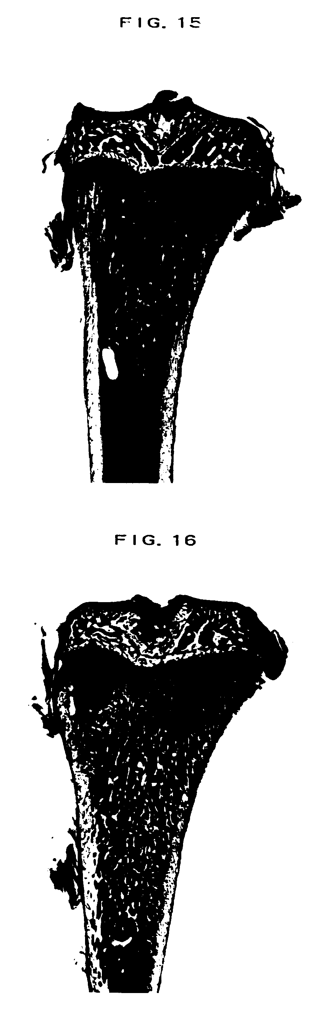

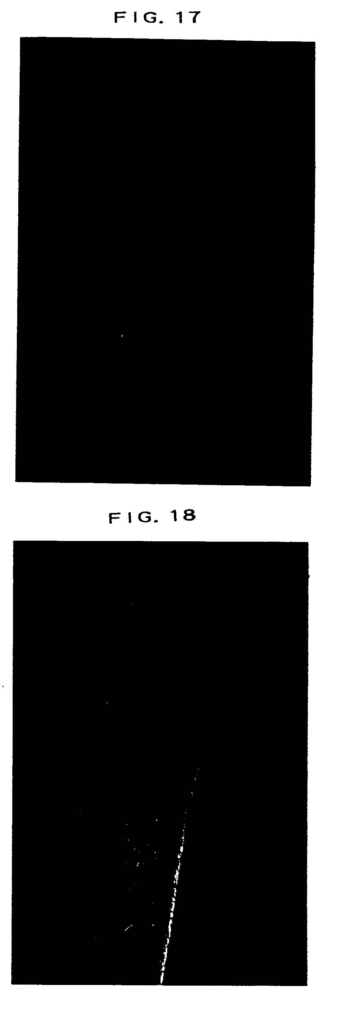

Light microscopy on the polished specimens revealed partial hypertrophy of the growth plate in the proximal portion of the tibia and marked proliferation of cancellous bone in the medulla from metaphysis to diaphysis in the 0.1 mg/kg administration group (see Figures 15 and 16). Since CMR specimen examination revealed the presence of a clear shadow image in almost the entire cancellous bone proliferated, the proliferated cancellous bone was identified as calcified bone (see Figures 17 and 18).

Details of Figures 15 through 18 are as follows:
Figure 15: Tibia, Jcl: Wistar rat, 8-week-old male, control group, VB stained, × 8.5, no abnormal findings.
Figure 16: Tibia, Jcl: Wistar rat, 8-week-old male, rhbFGF mutein CS23 0.1 mg/kg group, VB stained, × 8.5, showing marked proliferation of cancellous bone in the medulla of metaphysis and diaphysis and partial hypertrophy in the proximal growth plate.
Figure 17: Tibia, Jcl: Wistar rat, 8-week-old male, control group, CMR, × 7.5, no abnormal findings.
Figure 18: Tibia, Jcl: Wistar rat, 8-week-old male, rhbFGF mutein CS23 0.1 mg/kg group, CMR, × 7.5, showing marked proliferation of calcified cancellous bone from metaphysis to diaphysis.

### Example 7 Osteogenesis promoting action of rhbFGF mutein CS23 in aged rats

Four female rats (Jcl: Wistar) were dosed with recombinant human basic fibroblast growth factor (rhbFGF) mutein CS23 at a dose of 0.3 mg/kg/day by intravenous administration for 2 weeks starting at 71 weeks of age. One day after final administration, the animals were killed, and the femurs, sterna, tibias and lumbar vertebrae were fixed in 10% formalin, decalcified, embedded in paraffin, and sliced longitudinally to yield 4-micron sectionsl. After staining with hematoxylin-eosin (H-E), these sections were examined histopathologically to assess the effect on bone tissue. Animals in the control group were dosed with 50 mM citrate buffer (pH 7.0) in the same manner as of the treated groups.

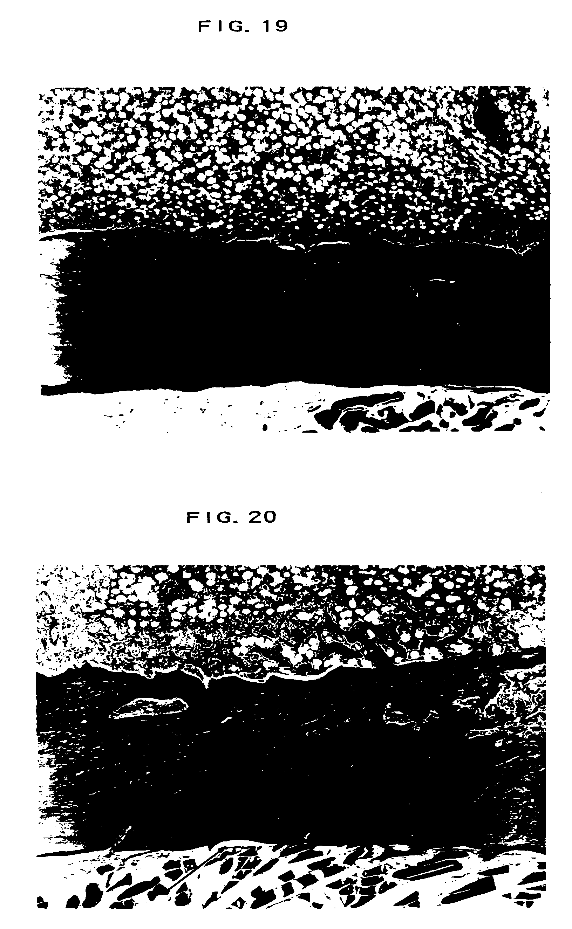

As a result, osteoblast proliferation and new bone formation were seen in all the bone tissues examined in the dosed group. This finding was obtained from the marrow cavity side of the metaphysis and diaphysis in the femurs (see figures 19 and 20) and tibias, and from the marrow cavity side of the central portion of the sterna and lumbar vertebrae. No bone formation occurred on the periosteal side (arrow).

Details of figures 19 and 20 are as follows:
Figure 19: Femur, Jcl: Wistar rat, 73-week-old femal, untreated control group, H-E stained, x 45, no abnormal findings.
Figure 20: Femur, Jcl: Wistar rat, 73-week-old female, rhbFGF mutein CS23 0.3 mg/kg group, H-E stained, x 45, showing new bone formation in the marrow cavity. No bone formation is seen in the periosteal side (arrow).

The above results that rhbFGF mutein CS23 has a potential to stimulate endosteal bone formation in aged animals indicate that the growth factor has a potential to stimulate endosteal bone formation irrespective of the animal age. This characteristic of the growth factor is suited for a prophylactic/therapeutic medicament for bone-loss diseases because such diseases are most popular among aged humans.

### Example 8 Production of injection

An aqueous solution (pH 7.4) containing 0.5 mg of rhbFGF mutein CS23, 10 mg of sucrose and 15 mg of sodium citrate per ml was prepared for a stable injection.

## Claims

1. Use of a cell growth factor composition to produce a medicament to provide a prophylactic or therapeutic action for the stimulation of endosteal bone formation in warm-blooded animals.

2. The use in accordance with claim 1, wherein the prophylactic or therapeutic action is applied to a bone-loss disease.

3. The use in accordance with claim 1, wherein the prophylactic or therapeutic action is applied to osteoporosis.

4. The use in accordance with the claim 1, wherein the cell growth factor is one having a molecular weight of about 5000 to about 25000.

5. The use in accordance with claim 1, wherein the cell growth factor is one belonging to the fibroblast growth factor (FGF) family.

6. The use in accordance with claim 5, wherein the FGF family comprises a mutein of human basic FGF.

7. The use in accordance with claim 6, wherein the mutein comprises a polypeptide where at least one constituent cysteine of human basic FGF is replaced by serine.

8. The use in accordance with claim 1, wherein the cell growth factor is an epithelial cell growth factor (EGF) or its mutein.

9. The use in accordance with claim 1, wherein the cell growth factor is hst-1 or its mutein where 1 to 27 continuous constituent amino acid residues are lacking from hst-1.

10. The use in accordance with claim 1, wherein a calcium agent is administered to the animal concomitantly with the cell growth factor.

## Patentansprüche

1. Verwendung einer Zellwachstumsfaktorzusammensetzung zur Herstellung eines Medikaments, um eine prophylaktische oder therapeutische Wirkung bei der Stimulierung der endostealen Knochenbildung bei Warmblütern zu erzielen.

2. Verwendung gemäß Anspruch 1, wobei die prophylaktische oder therapeutische Wirkung auf eine Knochenschwundkrankheit angewandt wird.

3. Verwendung gemäß Anspruch 1, wobei die prophylaktische oder therapeutische Wirkung auf Osteoporose angewandt wird.

4. Verwendung gemäß Anspruch 1, wobei der Zellwachstumsfaktor ein Molekulargewicht von etwa 5000 bis etwa 25 000 hat.

5. Verwendung gemäß Anspruch 1, wobei der Zellwachstumsfaktor zu der Familie der Fibroblastenwachstumsfaktoren (FGF) gehört.

6. Verwendung gemäß Anspruch 5, wobei die FGF-Familie ein Mutein von basischem Human-FGF umfaßt.

7. Verwendung gemäß Anspruch 6, wobei das Mutein ein Polypeptid umfaßt, bei dem wenigstens ein Cysteinbestandteil von basischem Human-FGF durch Serin ersetzt ist.

8. Verwendung gemäß Anspruch 1, wobei der Zellwachstumsfaktor ein Epithelzellenwachstumsfaktor (EGF) oder ein Mutein davon ist.

9. Verwendung gemäß Anspruch 1, wobei der Zellwachstumsfaktor hst-1 oder ein Mutein davon, bei dem 1 bis 27 aufeinanderfolgende konstituierende Aminosäurereste von hst-1 fehlen, ist.

10. Verwendung gemäß Anspruch 1, wobei dem Tier gleichzeitig mit dem Zellwachstumsfaktor ein Calciummittel verabreicht wird.

## Revendications

1. Emploi d'une composition de facteur de croissance cellulaire pour la production d'un médicament offrant une action prophylactique ou thérapeutique de stimulation, chez les animaux à sang chaud, de la formation de tissu osseux au niveau de l'endostéum.

2. Emploi conforme à la revendication 1, dans lequel l'action prophylactique ou thérapeutique combat une maladie où il y a perte de tissu osseux.

3. Emploi conforme à la revendication 1, dans lequel l'action prophylactique ou thérapeutique combat l'ostéoporose.

4. Emploi conforme à la revendication 1, dans lequel la masse moléculaire du facteur de croissance cellulaire vaut d'environ 5 000 à environ 25 000.

5. Emploi conforme à la revendication 1, dans lequel le facteur de croissance cellulaire appartient à la famille des facteurs de croissance des fibroblastes (FGF).

6. Emploi conforme à la revendication 5, dans lequel la famille des FGF comprend une mutéine de FGF basique humain.

7. Emploi conforme à la revendication 6, dans lequel la mutéine est un polypeptide où un résidu de sérine remplace au moins l'un des résidus de cystéine constitutifs du FGF basique humain.

8. Emploi conforme à la revendication 1, dans lequel le facteur de croissance cellulaire est un facteur de croissance de cellules épithéliales (EGF) ou une mutéine d'un tel facteur.

9. Emploi conforme à la revendication 1, dans lequel le facteur de croissance cellulaire est hst-1 ou une mutéine de celui-ci, à laquelle il manque de 1 à 27 résidus d'acide aminé constitutifs successifs du hst-1.

10. Emploi conforme à la revendication 1, dans lequel on administre à l'animal, conjointement avec le facteur de croissance cellulaire, un agent apportant du calcium.
